(19) Europäisches Patentamt
European Patent Office
Office européen des brevets

(11) EP 1 124 532 B1

(12) EUROPEAN PATENT SPECIFICATION

(45) Date of publication and mention of the grant of the patent:
**19.03.2003 Bulletin 2003/12**

(21) Application number: **99971304.3**

(22) Date of filing: **13.10.1999**

(51) Int Cl.[7]: **A61K 7/48**, A61K 7/00

(86) International application number:
**PCT/US99/23504**

(87) International publication number:
**WO 00/025738 (11.05.2000 Gazette 2000/19)**

# (54) TOPICAL VITAMIN COMPOSITION

VITAMINZUSAMMENSETZUNG ZUR TOPISCHEN ANWENDUNG

COMPOSITION TOPIQUE VITAMINEE

(84) Designated Contracting States:
**AT BE CH CY DE DK ES FI FR GB GR IE IT LI LU MC NL PT SE**
Designated Extension States:
**RO**

(30) Priority: **30.10.1998 US 182932**

(43) Date of publication of application:
**22.08.2001 Bulletin 2001/34**

(73) Proprietor: **Colgate-Palmolive Company**
**New York, N.Y. 10022 (US)**

(72) Inventors:
- **SOLIMAN, Nadia**
**East Brunswick, NJ 08816 (US)**
- **NABI, Zeenat, F.**
**Cranbury, NJ 08512 (US)**

(74) Representative: **Prins, Adrianus Willem et al**
**Vereenigde,**
**Nieuwe Parklaan 97**
**2587 BN Den Haag (NL)**

(56) References cited:
**WO-A-99/13829**
**US-A- 5 565 189**
**US-A- 5 573 756**

- **DATABASE WPI Section Ch, Week 199150 Derwent Publications Ltd., London, GB; Class D21, AN 1991-365786 XP002129434 & JP 03 246215 A (LION CORP), 1 November 1991 (1991-11-01)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 124 532 B1

## Description

### Background of the Invention

Skin is continuously subjected to extremes of environmental stress and air pollution. Such insults can bring about generation of free radicals in skin which are a significant contributor to skin cell damage, premature aging of skin, skin cancer and the like. These free radicals are produced naturally in the body by such means as metabolic processes, lifestyle excesses, for example, exercise and the like as well as environmental insults including exposure to ozone, heavy metals, halogenated hydrocarbons, ionization radiation and cigarette smoke.

It is generally accepted that materials having an antioxidant effect play a significant role against free radical induced skin damage. Key among these antioxidants are compounds commonly referred to as vitamins, particularly vitamins E and A. Since the human body is unable to synthesize its own Vitamin E it must be provided by extraneous sources, such as food and supplements. Recently, there have been attempts to deliver the vitamins to the skin from various skin cleansing and treatment compositions.

It has now been found that various vitamins and their precursors can be effectively delivered to the skin through the use of an aqueous liquid composition having surfactant therein which also has included therein a small amount of an alkyl or alkenyl mono or diethanolamide. Such amide(s) effectively solubilize the vitamin or vitamin derivative allowing them to be effectively delivered to the skin from the composition.

US-A-5 565 189 and US-A-5 573 756 both disclose compositions comprising components (a), (b) and (c) of the present invention, but differ in the amount of component (c).

### Summary of the Invention

In accordance with the invention, there is an aqueous liquid composition comprising:

(a) at least 1 wt% of a surfactant, or mixtures thereof;
(b) 0.01 to 2 wt% of a material selected from the group consisting of vitamin E, vitamin C, vitamin A, a precursor of any of these said vitamins which is converted to the respective vitamin E, C and A when said precursor is contacted with skin, and mixtures thereof; and
(c) 0.5 to 2 wt% of an alkyl or alkenyl mono or di alcohol amide or mixtures thereof, said alkyl or alkenyl having from 8 to 20 carbon atoms, inclusive and said alcohol having from about one to three carbon atoms.

### Detailed Description of the Invention

There must be at least one surfactant present in the composition. The surfactant can be anionic, nonionic, amphoteric, or cationic, preferably anionic. Soap, a long chain alkyl or alkenyl, branched or normal carboxylic acid salt such as sodium, potassium, ammonium or substituted ammonium salt, can be present in the composition as an example of an anionic surfactant. Exemplary of long chain alkyl or alkenyl are from 8 to 22 carbon atoms in length, specifically 10 to 20 carbon atoms in length, more specifically alkyl and most specifically normal, or normal with little branching. Small quantities of olefinic bond(s) may be present in the predominantly alkyl sections, particularly if the source of the "alkyl" group is obtained from a natural product such as tallow, coconut oil and the like. Because of its potential harshness soap is not a preferred surfactant and can be omitted from the composition.

Other surfactants can be present in the composition as well. Examples of such surfactants are the anionic, amphoteric, nonionic and cationic surfactants. Examples of anionic surfactants include but are not limited to soaps, alkyl sulfates, anionic acyl sarcosinates, methyl acyl taurates, N-acyl glutamates, acyl isethionates, alkyl sulfosuccinates, alkyl phosphate esters, ethoxylated alkyl phosphate esters, trideceth sulfates, protein condensates, mixtures of ethoxylated alkyl sulfates and the like.

Alkyl chains for these surfactants are $C_8$-$C_{22}$, preferably $C_{10}$-$C_{18}$, more preferably $C_{12}$-$C_{14}$.

Anionic non-soap surfactants can be exemplified by the alkali metal salts of organic sulfate having in their molecular structure an alkyl radical containing from 8 to 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$-$C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and

others known in the art.

Zwitterionic surfactants can be exemplified by those which can be broadly described as derivatives of aliphatic quaternary ammonium, phosphonium, and sulfonium compounds, in which the aliphatic radicals can be straight chain or branched and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

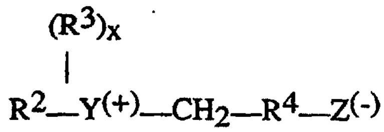

wherein $R^2$ contains an alkyl, alkenyl, or hydroxy alkyl radical of from about 8 to about 18 carbon atoms, from 0 to about 10 ethylene oxide moieties and from 0 to I glyceryl moiety; Y is selected from the group consisting of nitrogen, phosphorus, and sulfur atoms; R3 is an alkyl or monohydroxyalkyl group containing 1 to about 3 carbon atoms; X is I when Y is a sulfur atom and 2 when Y is a nitrogen or phosphorus atom, $R^4$ is an alkylene or hydroxyalkylene of from 0 to about 4 carbon atoms and Z is a radical selected from the group consisting of carboxylate, sulfonate, sulfate, phosphonate, and phosphate groups.

Examples include: 4-[N,N-di(2-hydroxyethyl)-N-octadecylammonio]-butane-1-carboxylate; 5-[S-3-hydroxypropyl-S-hexadecylsulfonio] -3 hydroxypentane-1-sulfate; 3-[P,P-P-diethyl-P 3,6,9 trioxatetradecyl- phosphonio]-2-hydroxypropane-1-phosphate; 3-[N,N-dipropyl-N-3 dodecoxy-2-hydroxypropylammonio]-propane-l-phosphonate; 3-(N, N-dimethyl-N-hexadecylammonio) propane-1-sulfonate; 3-(N,N-dimethyl-N-hexadecylammonio)-2-hydroxypropane-1-sulfonate; 4-(N,N-di(2-hydroxyethyl)-N-(2 hydroxydodecyl) ammonio]-butane-1-carboxylate; 3-[S-ethyl-S-(3-dodecoxy-2-hydroxypropyl)sulfonio]-propane-1-phosphate; 3-(P,P-dimethyl-P-dodecylphosphonio)-propane-1-phosphonate; and 5-[N,N-di(3-hydroxypropyl)-N-hexadecylammonio]-2-hydroxy-pentane-1-sulfate.

Examples of amphoteric surfactants which can be used in the compositions of the present invention are those which can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyltaurines, such as the one prepared by reacting dodecylamine with sodium isethionate according to the teaching of U.S. Patent No.2,658,072, N-higher alkyl aspartic acids, such as those produced according to the teaching of U.S. Patent No. 2,438,091, and the products sold under the trade name "Miranol" and described in U.S. Patent No. 2,528,378. Other amphoterics such as betaines are also useful in the present composition.

Examples of betaines useful herein include the high alkyl betaines such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl) carboxymethyl betaine, oleyl dimethyl gamma-carboxypropyl betaine, lauryl bis-(2-hydro-xypropyl) alpha-carboxyethyl betaine, etc. The sulfobetaines may be represented by coco dimethyl sulfopropyl betaine, stearyl dimethyl sulfopropyl betaine, amido betaines, amidosulfobetaines, and the like.

Many cationic surfactants are known to the art. By way of example, the following may be mentioned:

- stearyldimenthylbenzyl ammonium chloride;
- dodecyltrimethylammonium chloride;
- nonylbenzylethyldimethyl ammonium nitrate;
- tetradecylpyridinium bromide;
- laurylpyridinium chloride;
- cetylpyridinium chloride
- laurylpyridinium chloride;
- laurylisoquinolium bromide;
- ditallow(Hydrogenated)dimethyl ammonium chloride;
- dilauryldimethyl ammonium chloride; and
- stearalkonium chloride.

Additional cationic surfactants are disclosed in USP 4,303,543 see column 4, lines 58 and column 5, lines 1-42. Also see CTFA Cosmetic Ingredient Dictionary, 4th Edition 1991, pages 509-514 for various long chain alkyl

cationic surfactants.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerized propylene, diisobutylene, octane, or nonane, for example.
2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from about 40% to about 80% polyoxyethylene by weight and having a molecular weight of from about 5,000 to about 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.
3. The condensation product of aliphatic alcohols having from 8 to 18 carbon atoms, in either straight chain or branched chain configuration with ethylene oxide, e.g., a coconut alcohol ethylene oxide condensate having from 10 to 30 moles of ethylene oxide per mole of coconut alcohol, the coconut alcohol fraction having from 10 to 14 carbon atoms. Other ethylene oxide condensation products are ethoxylated fatty acid esters of polyhydric alcohols (e.g., Tween 20-polyoxyethylene (20) sorbitan monolaurate).
4. Long chain tertiary amine oxides corresponding to the following general formula:

$$R_1R_2R_3N \rightarrow 0$$

wherein $R_1$ contains an alkyl, alkenyl or monohydroxy alkyl radical of from about 8 to about 18 carbon atoms, from 0 to about 10 ethylene oxide moieties, and from 0 to 1 glyceryl moiety, and, $R_2$ and $R_3$ contain from 1 to about 3 carbon atoms and from 0 to about 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyl-di(2-hydroxyethyl) amine oxide, dimethyloctylamine oxide, dimethyldecylamine oxide, dimethyltetradecylamine oxide, 3,6,9 trioxaheptadecyldiethylamine oxide, di(2-hydroxyethyl)-tetradecylamine oxide, 2-dodecoxyethyldimethylamine oxide, 3-dodecoxy-2-hydroxypropyldi(3-hydroxypropyl)amine oxide, dimethylhexadecylamine oxide.
5. Long chain tertiary phosphine oxides corresponding to the following general formula:

$$RR'R''P \rightarrow 0$$

wherein R contains an alkyl, alkenyl or monohydroxyalkyl radical ranging from 8 to 20 carbon atoms in chain length, from 0 to about 10 ethylene oxide moieties and from 0 to 1 glyceryl moiety and R' and R" are each alkyl or monohydroxyalkyl groups containing from 1 to 3 carbon atoms. The arrow in the formula is a conventional representation of a semipolar bond. Examples of suitable phosphine oxides are: dodecyldimethylphosphine oxide, tetradecylmethylethylphosphine oxide, 3,6,9-trioxaoctadecyldimethylphosphine oxide, cetyldimethylphosphine oxide, 3-dodecoxy-2-hydroxypropyldi(2-hydroxyethyl) phosphine oxide stearyldimethylphosphine oxide, cetylethyl propylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl)phosphine oxide, tetradecylmethyl-2-hydroxypropylphosphine oxide, oleyldimethylphosphine oxide, 2-hydroxydodecyldimethylphosphine oxide.
6. Long chain dialkyl sulfoxides containing one short chain alkyl or hydroxy alkyl radical of 1 to about 3 carbon atoms (usually methyl) and one long hydrophobic chain which contain alkyl, alkenyl, hydroxy alkyl, or keto alkyl radicals containing from about 8 to about 20 carbon atoms, from 0 to about 10 ethylene oxide moieties and from 0 to 1 glyceryl moiety. Examples include: octadecyl methyl sulfoxide, 2-ketotridecyl methyl sulfoxide, 3,6,9-trioxaoctadecyl 2-hydroxyethyl sulfoxide, dodecyl methyl sulfoxide, oleyl 3-hydroxypropyl sulfoxide, tetradecyl methyl sulfoxide, 3 methoxytridecylmethyl sulfoxide, 3-hydroxytridecyl methyl sulfoxide, 3-hydroxy-4-dodecoxybutyl methyl sulfoxide.
7. Alkylated polyglycosides wherein the alkyl group is from 8 to 20 carbon atoms, preferably 10 to 18 carbon atoms

and the degree of polymerization of the glycoside is from 1 to 3, preferably 1.3 to 2.0.

The quantity of surfactant to be employed in the composition is not unduly significant. It should generally be sufficient to exert a cleansing effect upon skin. Generally, a minimum of 1 wt% of the composition can be a surfactant or mixtures thereof. Preferably 2,3,4 or 5 wt% can be employed as a minimum. A maximum of 30 wt% of the composition can be a surfactant or mixture thereof; preferably a maximum of 25, 20 or 18 wt% can be employed. Generally at least 20 wt% of surfactant present is an anionic surfactant or mixture thereof, preferably a mild anionic surfactant. Usually, the anionic surfactant or mixture thereof is at least 30 or 40 wt% of the surfactant present. Soap need not be present. Any specific surfactant or group of surfactants, i.e. amphoteric, zwitterionic, nonionic and cationic can individually or in groups be omitted.

The vitamins which have antioxidant effects include E, C and A. Generally, it is preferred to use a derivative of the actual vitamin since the vitamins per se are somewhat unstable in comparison to various derivatives, particularly in an aqueous liquid composition. When in contact with skin, these vitamin derivatives are converted to the vitamin per se so it can exert its antioxidant effect. For example, vitamin E, a tocopherol, is utilized in compositions preferably as the methyl ester which is then bioconverted to vitamin E in the skin. In a similar manner, vitamin A is utilized in the composition as a palmitate ester, for example, which is then bioconverted by skin to its antioxidant effective moiety, vitamin A. Any precursor which is converted by a skin containing system to the active vitamin antioxidant can be employed in the composition. For example, esters of the vitamins can be employed as effective precursors. Examples of effective esters are those having 1 to 20 carbon atoms, for example, the methyl, propyl, hexyl, decyl, lauryl, palmityl and behenyl ester of the vitamin such as the methyl ester of vitamin E or the palmitate ester of vitamin A. Of the actual vitamin the alpha tocopherol compound is preferred as Vitamin E. Similar precursors can be used for Vitamin C.

The vitamin or vitamin precursor and mixture thereof can be present in the composition in quantities of at least 0.01 wt%, or generally 0.1, 0.2, or 0.5 wt%. Lesser amount of vitamin A or precursor can be present as opposed to vitamin E or precursor. Maximum quantities of vitamin or precursor generally do not exceed 2.0 wt% of the composition, or generally 1.5 or 1.0 wt% of the composition.

The proper integration of the vitamin or precursor in the liquid aqueous composition is highly significant for its performance as an antioxidant skin protecting material. The vitamin or precursor must be sufficiently solubilized in the composition so as to be compatible, not separate into a second phase, and not cause unfavorable interactions amongst the other composition components at all or to any great extent. However, it must still be available for ready deposition upon the skin during the time the composition is in contact with the skin. Although various materials have been tried and do not meet all these criteria, a specific group of materials in appropriate concentration ranges are effective. The long chain alkyl or alkenyl mono and di alcohol amides are surprisingly effective in particular a diethanol amide or mono ethanol amide. These materials at a concentration generally below 2 wt%, preferably below 1.5 wt% of the composition solubilize the vitamin or precursor, particularly the methyl ester of Vitamin E and the palmitate ester of Vitamin A. They are compatible with the overall composition and permit deposition of effective levels of vitamins or precursors on the skin. Any minimum level of amide which permits these occurrences can be employed. However, generally a minimum of 0.2 wt% of the liquid, aqueous composition, preferably a minimum of 0.5 wt% of the composition can be employed.

The composition can take the form of a "rinse-off" cleansing composition. It can also be applied as a "leave-on" cream or a lotion. The "rinse-off" composition is unusual since a significant quantity of vitamin can actually be deposited on the skin during the cleansing procedure. Such quantity can bring about significant suppression of the effects of an oxidant such as cumene hydroperoxide.

The rinse-off cleansing formulation can be prepared in the usual manner known to the art. However, it is preferable to add the amide with the vitamin or precursors prior to contact with the remainder of the composition.

The compositions of the invention are preferably clear to the eye after visual evaluation, particularly the rinse off composition. The viscosity of the compositions can vary from 1,000 to 60,000 mPas (centipoise). This is from a low viscosity rinse off composition to a high viscosity lotion or cream. With respect to a rinse off composition, the viscosity is generally from 1,000 to 15,000, preferably 2,000 to 12,000 mPas (centipoise) as measured at 25°C on a Brookfield RVTD viscometer using a spindle number 5 at 20 rpm. For the more viscous formulations, a T-bar "spindle" is employed, number C at 3 to 5 rpm. Generally, the compositions have a pH of 4.5 to 7, preferably a pH of 5 to 6.5 or 5 to 6.

A generalized preparation procedure is the following:

Charge water plus cationic polymer if present to the main vessel. Add surfactants and mix until well blended. Premix in a separate vessel cocodiethanolamide with vitamin or precursor until well blended. Add this pre-mix to the main vessel having the surfactants until well blended. Add minor components such as fragrance, preservative, viscosity and pH adjuster, and color or pearlizer to the main vessel and blend well.

Below are examples of the invention. These examples are intended to be illustrative of the broad concept of

the invention and not be unduly restrictive thereof. Comparison examples are also included to demonstrate the unusual character and particular advantageousness of the invention. It should be noted that one particular significant advantage of compositions of the invention is that the lather generated by this composition is not significantly different than the lather generated by the same composition but without the presence of the oily vitamin and/or precursor. Oils are generally known to retard the lathering caused by surfactants. These results are observed in an in vitro laboratory test and conformed by a trained human sensory panel.

Example 1

Common cleansing bases having 8.4 wt% sodium laureth-2-sulfate, 3 wt% cocoamidopropylbetaine, 1% vitamin E-acetate, a specified amount of solubilizing material and a specified amount of polyquat 7 (PQ7) are prepared. CDEA is cocodiethanolamide, and Tween 80 is polysorbate 80. ("w/" means "with"). The remainder of each composition is essentially water. The composition is prepared and visually evaluated after 24 to 72 hours. Below are the results.

Table 1

| Composition | Wt% PQ-7 | Wt% Solubilizer | Observation |
|---|---|---|---|
| | | | |
| A (1.125 wt% APG) | 0 | 0 | off-white floating vitamin layer |
| B | 0 | 2.25% Tween 80 | opaque off-white oil droplets |
| C | 0 | 4% Tween 80 | clear, no viscosity |
| D | .04 | 3% Laureth-7 | opaque, with off-white rim, no viscosity |
| E | .04 | 2% Laureth-7<br>2% Tween 80 | opaque, hazy no viscosity |
| F | 0.2 | 3% CDEA<br>1% Tween 80 | clear, 640 cps viscosity w/salt |
| G | 0.2 | 4% CDEA | hazy, 3400 cps viscosity w/salt |
| H | 0.2 | 3% CDEA | clear, 1200 cps viscosity w/salt |
| I | .04 | 2% CDEA | opaque, w/off-white oil droplets |
| J | 0.2 | 2% CDEA | clear, 160 cps viscosity w/salt |
| K | .04 | 2% Tween 80<br>1% CDEA | clear, no viscosity |
| L | 0.2 | 3% CDEA<br>no vitamins | clear 9800 cps viscosity without salt |
| M | 0 | 1% CDEA | clear w/off-white floating layer |
| N | 0.2 | 1% CDEA | clear, 300 cps viscosity w/salt |

As shown by the data in Table 1, a number of potential solubilizer systems are tested and only a few give satisfactory visual aesthetics and viscosity buildup for even further consideration for actual deposition studies. These are studied below.

Example 2

The compositions C, H, J and N from Table 1 are tested for deposition of the precursor vitamin E acetate onto pig skin using the following procedure.

Porcine (pig) skin samples are cut (3.18x3.18 cm) (1.25x1.25 inch) and stored at -20°C freezer. On the day of experiment, the pig skins are thawed for 45 minutes (water is sprayed from a squirt bottle periodically to prevent dehydration). The skin samples are set on a deposition chamber as described in U.S. Patent 4,836,014 (P. Hilliard) issued 1989 and the screws tightened firmly. The shower gel (100 µl) as applied to each skin sample, rubbed gently with a glass rod for 15 seconds, and equilibrated for 45 seconds. The shower gel solution is then removed and the skin washed 10 times with water. Each time the chamber is filled up to the rim with water. The water is removed with a pipette tip attached to a vacuum. The deposited materials are extracted with 1 ml ethanol for one minute. The process

is repeated 3 times. The pooled ethanol extracts are evaporated to dryness under $N_2$ and stored at -20°C. To analyze the vitamin contents by HPLC the dried material is resuspended in a 0.5 ml solution of methanol:isopropanol:butanol (70:20:10).

The HPLC conditions were:

Column = 100 mm Phenomenex Ultracarb 5u-ODS 20
Mobile phase = 75 methanol/20 isopropanol/5 butanol
Flow rate 0.75 ml/min.
Wave length was 288 mm.
Under these conditions, retention times for the vitamins were:
Vitamin E =5.0 and Vitamin E acetate = 7.0 min. approximately.

| Composition | Solubilizer | Deposition of Vitamin E Acetate μg/$cm^2$ skin |
|---|---|---|
| C | 4% Tween 80 | 0.1 |
| H | 3% CDEA | 0.39 |
| J | 2% CDEA | 0.49 |
| N | 1% CDEA | 0.62 |

As shown by this data, higher quantities of solubilizers have a negative effect on provitamin deposition. The 1 wt% CDEA provided substantially more deposition than the 3% CDEA with the 2% CDEA falling between the two levels.

Example 3

In general the incorporation of provitamin A palmitate has an adverse effect on product viscosity as well as color, particularly over aging. Therefore, lower quantities of Vitamin A palmitate have been effectively employed, 0.01 wt% of the composition. The best balance of properties, including visual and viscosity occurs when ammonium lauryl sulfate (ALS) is employed rather than sodium laureth-2-sulfate (SLES) in the composition. A specific composition has 11.2 wt% ammonium lauryl sulfate, 1.5 wt% cocoamidopropylbetaine (CAPB), 1 wt% CDEA, 1 wt% vitamin E acetate, 0.01 wt% vitamin A palmitate, and 0.12 wt% polyquat-7. Such composition is clear, essentially colorless and has a viscosity of 4,000 mPas (cps). The substitution of ALS for SLES is found to increase viscosity 2,000-4,000 mPas (cps) depending on the ratio of ALS/CAPB. This substitution at certain ratio(s) does not require any additional thickeners for the composition, depending upon desired viscosity. Further, more provitamins show better stability in the ALS/CAPB, even without the presence of polyquat-7.

Example 4

A further preferred composition comprises 11.2 wt% ammonium lauryl sulfate, 1.5 wt% CAPB, 1 wt% CDEA, 1 wt% vitamin E acetate, 0.12 wt% polyquat 7, 0.01 wt% vitamin A palmitate with tocopherol, 0.9 wt% of a mixture of glycol distearate, laureth-4 and CAPB, 0.4 wt% glycerin, preservative, salt, citric acid and the balance water. The composition has a pH of about 5.5 and a viscosity at room temperature using a Brookfield RVTD viscometer with a number 5 spindle and 20 rpm of about 4,500 mPas (cps.)

In vitro and In vivo test protocols and results showing efficacy of vitamin and vitamin precursors in protecting against free radical attack on skin (antioxidant activity) are conducted.

In Vitro

The antioxidant benefits of various formulations are measured by applying the compositions to skin equivalent tissues (Epiderm™) purchased from MatTek. After 1 minute of contact, skin samples are rinsed with phosphate buffered saline, and incubated in a maintenance medium for 3 hours to allow the bioconversion of the provitamin. Cumene hydroperoxide was added to the stratum corneum (sc) surface. Cumene peroxide-induced cell cytotoxicity and the protection by the formulations are analyzed by MTT or Alamar blue assay.

The MTT assay uses 3-[4,5-dimethylthiazol-2-yl]-2,5-diphenyltetrazolium bromide (MTT-reduction assay). MTT solution is prepared as 0.5 mg/ml in phosphate buffered saline just before use and filtered through a 0.22 μm

filter. MatTek EpiDerm skins are placed in 24 well tissue culture plates and 500 µl of the MTT solutions are added to each well and incubated at 37°C for 4 hours. The cell survivability is analyzed by measuring the ability of the cells mitochondria to reduce the colorless MTT to a colored formazan(crystals) end product. The reaction is terminated by the removal of the MTT solution. Addition of 500 µl of 0.04N HCl is isopropanol to each well is used to dissolve the intracellular MTT formazan crystals. The contents of each well are mixed gently in an orbital shaker at room temperature for 2 hours and the absorbency at 570 nm is measured by an ELISA reader (Titertek, Multiscan, MC).

<u>In Vivo</u>

To measure the antioxidant benefits of the formulations, human forearms are washed with the formulation for 1 minute (30 second lather, 30 second waiting, and 15 second rinse). Three hours after the wash, skin samples are collected with tapes. Alkyl peroxide (cumene hydroperoxide) was added to the stratum corneum side of the tape. Neutralizing efficacy or antioxidant benefits of formulations are identified by measuring the remaining cumene hydroperoxide on the stratum corneum. This is measured by LPO (lipid peroxide) assay kit (Kamiya Biomedical Company, Seattle, WA) utilizing the directions therein.

The LPO assay is performed using Determiner® LPO kit (Kamiya Biomedical Company, WA). The kit utilizes hemoglobin to catalyze the transfer of an oxidizing equivalent from the lipid hydroperoxide to a colorless derivative of methylene blue, forming a colored compound which can be measured spectrophotometrically. A 20 µl sample of tissue extract is added to a microfuge tube containing 250µl of reagent-1; after vortex mixing, the solution is incubated for 5 minutes at 30°C. 500µl of Reagent-2 is added. The reaction mixture is vortex mixed again and incubated at 30°C for 30 minutes. The samples are then centrifuged at 800 x g for 6 minutes to remove tissue debris. The absorbency of the supernatant is measured at 674 nm in a spectrophotometer.

| Results | | |
|---|---|---|
| **Sample** | **% Protection Against Oxidant** | |
| | **In Vitro** | **In Vivo** |
| Example 3 without vitamin or vitamin precursor | 0 | 5 |
| Example 3 | 29 | 30 |
| Example 3 with Vitamin E and no precursor | 79 | 46 |

## Claims

1. An aqueous liquid composition comprising:

   (a) at least 1 wt% of a surfactant and mixtures thereof;
   (b) 0.01 to 2 wt% of a material selected from the group consisting of vitamin E, vitamin C, vitamin A, a precursor of any of these said vitamins which is converted to the respective vitamin E, C and A when said precursor is contacted with skin, and mixtures thereof; and
   (c) 0.5 to 2 wt% of an alkyl or alkenyl mono or di alcohol amide, said alkyl or alkenyl having from 8 to 20 carbon atoms, inclusive and said alcohol having from about one to three carbon atoms, and mixtures thereof.

2. The composition in accordance with claim 1 wherein the surfactant is anionic.

3. The composition in accordance with claim 1 wherein at least 2 wt% of the composition is a surfactant.

4. The composition in accordance with claim 1 wherein the material is vitamin A.

5. The composition in accordance with claim 1 wherein the material is a vitamin A precursor which is converted to vitamin A when contacted with skin.

6. The composition in accordance with claim 1 wherein the material is vitamin E.

7. The composition in accordance with claim 1 wherein the material is a vitamin E precursor which is converted to vitamin E when contacted with skin.

8. The composition in accordance with claim 1 wherein component (c) is a diethanol amide.

9. The composition in accordance with claim 1 wherein component (c) is a monoethanol amide.

10. The composition in accordance with claim 5 wherein the material is the palmitate ester of vitamin A.

11. The composition in accordance with claim 7 wherein the material is the methyl ester of vitamin E.

12. The composition in accordance with claim 11 wherein the material is the methyl ester of alpha tocopherol.

**Patentansprüche**

1. Wässrige flüssige Zusammensetzung, die

(a) mindestens 1 Gew.-% eines Tensids und Mischungen desselben,

(b) 0,01 bis 2 Gew.-% eines Materials ausgewählt aus der Gruppe bestehend aus Vitamin E, Vitamin C, Vitamin A, einem Vorläufer für irgendeines dieser Vitamine, der in das jeweilige Vitamin E, C und A umgewandelt wird, wenn der Vorläufer mit Haut in Kontakt gelangt, und Mischungen derselben, und

(c) 0,5 bis 2 Gew.-% eines Alkyl- oder Alkenylmono- oder -dialkoholamids umfasst, wobei das Alkyl oder Alkenyl 8 bis 20 Kohlenstoffatome aufweist, einschließlich, und der Alkohol etwa 1 bis 3 Kohlenstoffatome aufweist, und Mischungen derselben.

2. Zusammensetzung nach Anspruch 1, bei der das Tensid anionisch ist.

3. Zusammensetzung nach Anspruch 1, bei der mindestens 2 Gew.-% der Zusammensetzung Tensid sind.

4. Zusammensetzung nach Anspruch 1, bei der das Material Vitamin A ist.

5. Zusammensetzung nach Anspruch 1, bei der das Material ein vitamin A-Vorläufer ist, der bei Kontakt mit Haut in Vitamin A umgewandelt wird.

6. Zusammensetzung nach Anspruch 1, bei der das Material Vitamin E ist.

7. Zusammensetzung nach Anspruch 1, bei der das Material ein Vitamin E-Vorläufer ist, der bei Kontakt mit Haut in Vitamin E umgewandelt wird.

8. Zusammensetzung nach Anspruch 1, bei der Komponente (c) ein Diethanolamid ist.

9. Zusammensatzung nach Anspruch 1, bei der Komponente (c) ein Monoethanolamid ist.

10. Zusammensetzung nach Anspruch 5, bei der das Material der Palmitatester von Vitamin A ist.

11. Zusammensetzung nach Anspruch 7, bei der das Material der Methylester von Vitamin E ist.

12. Zusammensetzung nach Anspruch 11, bei der das Material der Methylester von $\alpha$-Tocopherol ist.

**Revendications**

1. Composition liquide aqueuse comprenant :

(a) au moins 1 % en poids d'un tensioactif et de mélanges de ce dernier ;
(b) de 0,01 à 2 % en poids total d'une substance choisie parmi le groupe constitué de la vitamine E, de la vitamine C, de la vitamine A, d'un précurseur de l'une quelconque desdites vitamines qui est converti en lesdites vitamines E, C et A respectives lorsque ledit précurseur est en contact avec la peau, et des mélanges

de ces derniers ; et
(c) de 0,5 à 2 % en poids total d'un alkyl ou alcényl d'alcool mono ou dialcolamide où, ledit groupement alkyl ou alcényl a, de façon inclusive, entre 8 et 20 atomes de carbone, et ledit alcool a environ entre 1 et 3 atomes de carbone, et des mélanges de ces derniers.

**2.** Composition selon la revendication 1 dans laquelle le tensioactif est anionique.

**3.** Composition selon la revendication 1 dans laquelle au moins 2 % en poids de la composition est un tensioactif.

**4.** Composition selon la revendication 1 dans laquelle la substance est la vitamine A.

**5.** Composition selon la revendication 1 dans laquelle la substance est un précurseur de vitamine A qui est converti en vitamine A lorsqu'il est mis en contact avec la peau.

**6.** Composition selon la revendication 1 dans laquelle la substance est la vitamine E.

**7.** Composition selon la revendication 1 dans laquelle la substance est un précurseur de vitamine E qui est converti en vitamine E lorsqu'il est mis en contact avec la peau.

**8.** Composition selon la revendication 1 dans laquelle le composant (c) est un diéthanolamide.

**9.** Composition selon la revendication 1 dans laquelle le composant (c) est un amide monoéthanolamide.

**10.** Composition selon la revendication 5 dans laquelle la substance est l'ester palmitique de la vitamine A.

**11.** Composition selon la revendication 7 dans laquelle la substance est l'ester méthylique de la vitamine E.

**12.** Composition selon la revendication 11 dans laquelle la substance est l'ester méthylique de l'alpha tocophérol.